# EUROPEAN PATENT SPECIFICATION

(11) **EP 1 679 306 B1**
(45) Date of publication and mention of the grant of the patent: **17.03.2010**
(21) Application number: 04792811.4
(22) Date of filing: 15.10.2004
(51) Int. Cl.: C07C 315/02, C07C 315/06, C07C 317/46, C07C 319/28, C07C 323/62

(54) **PROCESS FOR PRODUCING BICALUTAMIDE AND METHOD OF PURIFYING INTERMEDIATE THEREFOR**
VERFAHREN ZUR HERSTELLUNG VON BICALUTAMID UND VERFAHREN ZUR AUFREINIGUNG VON ZWISCHENPRODUKTEN DAFÜR
PROCEDE DE PRODUCTION DE BICALUTAMIDE ET PROCEDE DE PURIFICATION D'UN INTERMEDIAIRE

(30) Priority: 16.10.2003 JP 2003357038
(43) Date of publication of application: 12.07.2006
(73) Proprietor: Sumitomo Chemical Company, Limited, Tokyo 104-8260 (JP)
(72) Inventor: SHINTAKU, Tetsuya, Sanda-shi, Hyogo 669-1541 (JP); KATSURA, Tadashi, Toyonaka-shi, Osaka 560-0013 (JP); SUGI, Kiyoshi, Osaka-shi, Osaka 552-0003 (JP); ITAYA, Nobushige, Nishinomiya-shi, Hyogo 662-0841 (JP)
(74) Representative: Duckworth, Timothy John
(86) International application number: PCT/JP2004/015669
(87) International publication number: WO 2005/037777

(56) References cited:
- WO-A1-02/24638
- WO-A1-03/053920

## Description

### TECHNICAL FIELD

The present invention relates to a process for producing Bicalutamide.

### BACKGROUND ART

4'-Cyano-3-[(4-fluorophenyl)sulfonyl]-2-hydroxy-2-methyl-3'-trifluoromethylpro pionanilide (generic name: bicalutamide) is a pharmaceutical useful as a anticancer drug.

Generally speaking, pharmaceuticals are administered to humans in most cases, and commonly, amounts of impurities contained therein are strictly controlled. For example, it is necessary to examine and confirm that there is no problem that threatens safety, by analyzing, usually, the structure of the impurity when not less than 0.10% of an impurity is contained, and by studying the toxicity thereof when the amount is not less than 0.15%, in a pharmaceutical to be administered.

As indicated by the following scheme 1, a generally accepted process for producing
4'-cyano-3-[(4-fluorophenyl)sulfonyl]-2-hydroxy-2-methyl-3'-trifluoromethylpropionanili de (hereinafter, referred to as Compound (4)) is a process in which
4'-cyano-3-(4-fluorophenylthio)-2-hydroxy-2-methyl-3'-trifluoromethylpropionanilide (hereinafter, referred to as Compound (3)), obtained via
4-cyano-N-(2,3-epoxy-2-methylpropionyl)-3-trifluoromethylaniline (hereinafter, referred to as Compound (2)) starting form N-methacryloyl-4-cyano-3-trifluoromethylaniline (hereinafter, referred to as Compound (1)), is produced and Compound (3) is oxidized (for example, see WO 01/00608, WO 01 /28990, WO 02/24638, WO 03/053920 and Howard Tucker et al., J. Med. Chem., Vol. 31, 954-959 (1988)). However, it has been revealed that 4'-cyano-3-[(4-fluorophenyl)sulfonyl]-2-methyl-3'-trifluoromethylpropionanilide (hereinafter, referred to as Deoxy-Sulfonyl Compound) is by-produced as an impurity during oxidation. Additionally, it has also been revealed that separation and removal of Deoxy-Sulfonyl Compound from Compound (4) after oxidation reaction is difficult. Furthermore, the present inventors have found that the contamination with
Deoxy-Sulfonyl Compound as the impurity is attributed to
4'-cyano-3-(4-fluorophenylthio)-2-methyl-3'-trifluoromethylpropionanilide (hereinafter, referred to as Deoxy-Sulfide Compound) by-produced during the production of Compound (3).

One object of the present invention is to provide a process in which, in the production of Compound (4), the formation of hardly removable impurities contained in the final product are suppressed, and which is industrially excellent.

This and other objects will be apparent from the content of the description.

### DISCLOSURE OF THE INVENTION

As the result of extensive studies in view of the above problems, the present inventors have found the fact that the amount of Deoxy-Sulfonyl Compound contained in crystals of Compound (4) can be decreased to less than 0.10% (LC area percentage) based on the amount of Compound (4) by purifying crystals of Compound (3) by crystallization and subjecting the obtained pure crystals to the oxidation reaction when Deoxy-Sulfide Compound is contained in Compound (3) in an amount exceeding 0.06% (LC area percentage) based on the amount of Compound (3). The present invention was completed. Therefore, the invention is shown by the followings.
<1> A process for producing bicalutamide of the formula (4); 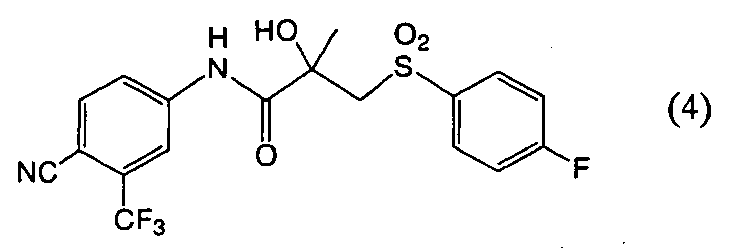 which comprises
   Step A comprising reacting Compound (1) of the formula (1); 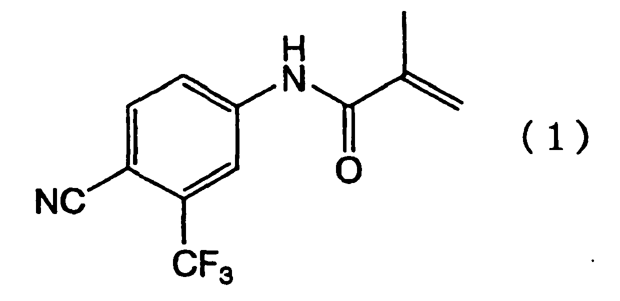 with peroxycarboxylic acid to obtain Compound (2) of the formula (2); 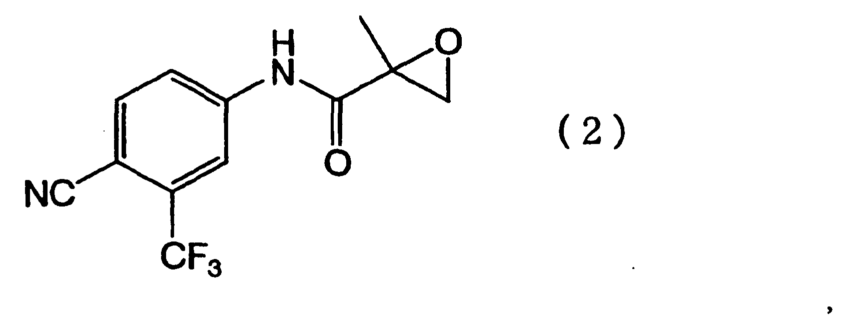
   Step B comprising reacting said Compound (2) with 4-fluorothiophenol to obtain crude crystals of Compound (3) of the formula (3); 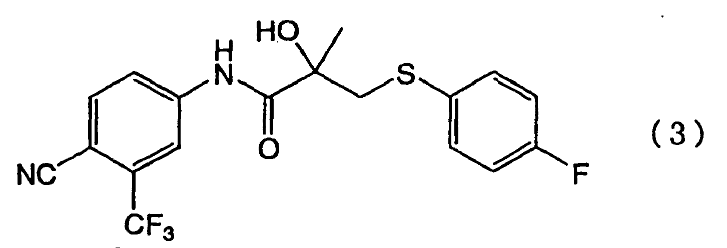 Comprising 0.06 to 0.5% of 4'-cyano-3-(4-fluorophenylthio)-2-methyl-3'-trifluoromethylpropionanilide, dissolving the crude crystals in a solvent and crystallizing to obtain purified crystals of Compound (3), and
   Step C comprising reacting Compound (3) and percarboxylic acid to obtain bicalutamide.
<2> The process according to <1>, wherein the solvent is the one containing toluene.
<3> The process according to <2>, wherein the solvent is the one consisting essentially of toluene.
<4> The process according to any of <1> to <3>, wherein seed crystals of purified Compound (3) are added before starting the crystallization.
<5> The process according to any of <1> to <4>, wherein the crystallization comprises maturing and cooling.
<6> The process according to <5>, wherein the maturing is conducted at a temperature of 55 ± 5°C.
<7> The process according to <5> or <6>, wherein the time of maturing is 1 to 2 hours.
<8> The process according to any of <5> to <7> wherein the cooling is conducted from 55°C to 10°C.
<9> The process according to any of <5> to <8>, wherein the speed of cooling is 5 to 15°C/hour.

### BEST MODE FOR CARRYING OUT THE INVENTION

The invention is described in detail by referring to the following scheme 1.

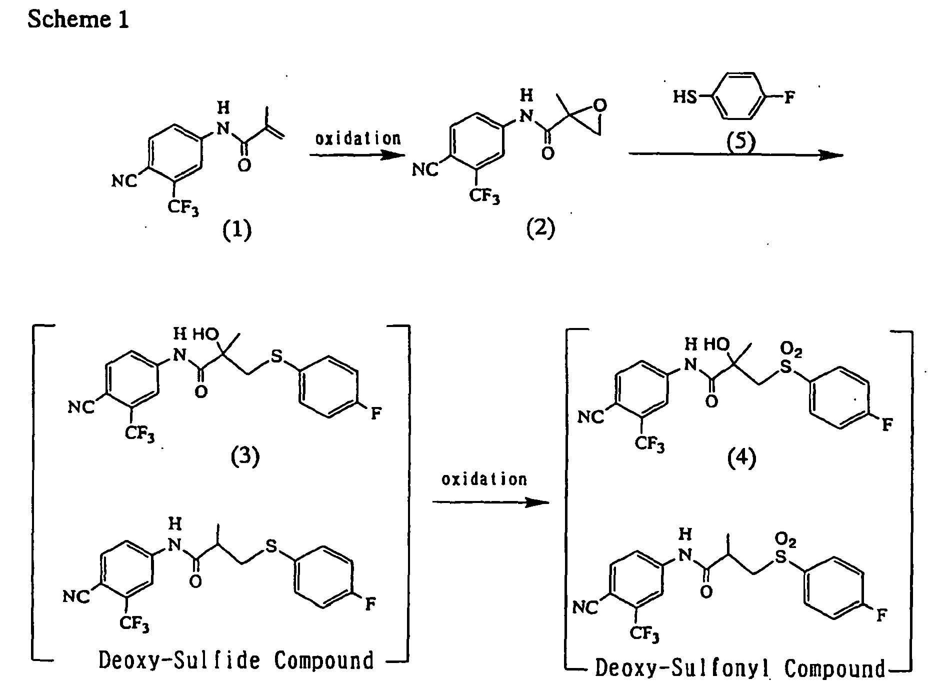

Compound (1) may be a commercially available product or a product synthesized according to a process known in the art (for example, WO 03/053920 and the like) or a similar process thereto. 4-Fluorothiophenol (hereinafter, referred to as Compound (5)) is also commercially available and this product can be used.

Compound (2) can be obtained by oxidizing Compound (1) (hereinafter, this step is referred to as Step A).

In Step A, a peroxycarboxylic acid as an oxidizing agent is added to Compound (1) in a suitable reaction solvent.

Examples of the peroxycarboxylic acid include m-chloroperbenzoic acid, mono-perphthalic acid and the like. From the viewpoints of safety and reactivity, mono-perphthalic acid is preferred.

Mono-perphthalic acid can easily be prepared, for example, by reacting phthalic anhydride with hydrogen peroxide.

Specifically, mono-perphthalic acid is prepared by mixing approximately equimolar amounts of phthalic anhydride and hydrogen peroxide in the presence of a base in a suitable solvent. Preferably, hydrogen peroxide is used in a small excess amount based on phthalic anhydride. Specifically, hydrogen peroxide is used in usually 1 to 1.5 moles, preferably 1 to 1.3 moles, based on 1 mole of phthalic anhydride.

Phthalic anhydride is preferred as a raw material for synthesis of mono-perphthalic acid because it is inexpensive, less hygroscopic and easily handled.

As hydrogen peroxide, it is preferred to use aqueous hydrogen peroxide solution from a viewpoint of easy handling. The aqueous hydrogen peroxide solution to be used has usually a concentration of 20 to 50% and preferably 30 to 35%. Aqueous hydrogen peroxide solution having a concentration of 30 to 35% is preferred because it is less explosively dangerous, commercially available and inexpensive.

Examples of the base include sodium carbonate, sodium hydrogen carbonate, potassium carbonate, potassium hydrogen carbonate and the like. From a viewpoint of economy, sodium carbonate is preferred.

The amount of the base to be used is usually 1 to 1.3 moles, preferably 1 to 1.2 moles, based on 1 mole of phthalic anhydride.

Examples of the solvent to be used include water or the like, amongst which deionized water is preferred from a viewpoint of the fact that it is free of metal which possibly exhibits catalytic activity in decomposing hydrogen peroxide, a viewpoint of solubility of hydrogen peroxide and a viewpoint of economy.

The amount of the solvent to be used is usually 2 to 5 ml, preferably 3 to 4 ml, based on 1 g of phthalic anhydride.

The reaction temperature is usually -5°C to +5°C and preferably -5°C to 0°C.

The reaction period varies depending on the reaction temperature and others but is usually 0.5 to 2 hours and preferably 0.5 to 0.8 hours.

After the reaction is terminated, the reaction system may be neutralized with an acid such as sulfuric acid (preferably 98% sulfuric acid) or the like, if necessary, and the product may be isolated and purified through the conventional post-treatment. Alternatively, the product may be used in the subsequent oxidation reaction (namely the above described Step A and Step C) without isolation and purification.

Examples of the solvent suitable for the reaction in Step A include, toluene, chlorobenzene, ethyl acetate and the like, ethyl acetate is preferred from a viewpoint of solubility of Compound (1).

The amount of the solvent to be used is usually 2 to 5 ml, preferably 2.5 to 4 ml, based on 1 g of Compound (1).

The amount of the peroxycarboxylic acid to be used is usually 1.5 to 3 moles, preferably 1.8 to 2.5 moles, based on 1 mole of Compound (1).

With regard to the method for adding the peroxycarboxylic acid, dropwise addition of a peroxycarboxylic acid solution is preferred from viewpoints of easiness of addition, safety and operability. When the peroxycarboxylic acid solution is added dropwise, the solution may be added in two or more divided portions.

Examples of solvent suitable for preparing the peroxycarboxylic acid solution include, ethyl acetate, ethers (for example, diethyl ether or the like) and the like, ethyl acetate is preferred from a viewpoint of safety. It is desirous to use the same solvent as the above solvent for reaction.

The amount of the solvent to be used for preparing the peroxycarboxylic acid solution is usually 3 to 10 ml, preferably 3.5 to 7 ml, based on 1 g of peroxycarboxylic acid.

When the peroxycarboxylic acid solution is added dropwise, the dropping rate depends on the concentration of the dropping solution, and the temperature of the dropping solution and drop-receiving solution, but is usually 1 to 4 ml/min and preferably 1.5 to 3 ml/min based on 1 g of Compound (1).

When the peroxycarboxylic acid solution is added dropwise, the temperature of the dropping solution is usually 0 to 35°C and preferably 10 to 30°C; the temperature of the drop-receiving solution is usually 20 to 60°C and preferably 40 to 55°C.

The reaction temperature is usually 20 to 60°C and preferably 45 to 55°C.

The reaction period varies depending on the reaction temperature and other reaction conditions but is usually 5 to 15 hours and preferably 6 to 9 hours.

After the reaction is completed, the reaction system may be changed to weakly basic (for example, pH 8) with a base such as potassium hydroxide, potassium carbonate or the like, if necessary, and the product may be isolated and purified through the conventional post-treatment.

Compound (3) can be obtained by reacting Compound (2) with Compound (5) (hereinafter, this step is referred to as Step B). The reaction in Step B is usually carried out in the presence of a base.

In Step B, examples of the base include, sodium hydride, sodium hydroxide, sodium carbonate, potassium hydroxide and the like. Sodium hydroxide is preferred from a viewpoint of economy. Sodium hydroxide is preferably aqueous sodium hydroxide solution from a viewpoint of easiness of handling. Commercially available aqueous sodium hydroxide solution can be used as it is or after dilution. The concentration of aqueous sodium hydroxide solution to be used is usually 5 to 20% by weight and preferably 15 to 20% by weight.

In Step B, a preferred method from a viewpoint of handling is a method in which a base is added in advance (preferably added by dropwise addition of a solution containing a base) to a solution in which Compound (5) is dissolved in a suitable reaction solvent, and Compound (2) is added (preferably added by dropwise addition of a solution containing Compound (2)) to the resulted mixture.

Examples of the solvent suitable for the reaction include polar solvents such as THF, tert-butanol and the like, and THF is preferred from a viewpoint of solubility of Compound (2).

The amount of the reaction solvent to be used is usually 1 to 40 ml, preferably 2 to 20 ml, based on 1 g of Compound (2).

The amount of the base to be used is usually 1 to 1.3 moles, preferably 1 to 1.2 moles, based on 1 mole of 4-fluorothiophenol.

The temperature for adding the base is usually 0 to 30°C and preferably 0 to 20°C.

The temperature for adding Compound (2) is usually 0 to 15°C and preferably 0 to 10°C.

When Compound (2) is added dropwise in the form of a solution, examples of the solvent includes aprotic solvents such as THF and the like, and THF is preferred from a viewpoint of solubility of Compound (2). It is desirous to use the same solvent as the above solvent for reaction. The amount of the solvent to be used is usually 1 to 10 ml, preferably 2 to 6 ml, based on 1 g of Compound (2).

The reaction temperature is usually 0 to 30°C and preferably 0 to 20°C.

The reaction period varies depending on the reaction temperature and other reaction conditions but is usually 1 to 20 hours and preferably 2 to 15 hours.

After the reaction is terminated, crude crystals of Compound (3) comprising 0.06 to 0.5% of Deoxy-Sulfide Compound are obtained by applying conventional treatments for isolation such as washing with an alkali, washing with an acid, evaporation of the solvent or the like.

In Step B, it has been found that Deoxy-Sulfide Compound was produced by the reaction of unreacted Compound (1) and Compound (5). This fact has been first found by the present inventors.

In addition, it has been found that Deoxy-Sulfonyl Compound could be derived from Compound (3).

Consequently, even when Compound (3) is synthesized from Compound (1) through Step A and Step B and the obtained compound (3) does not contain Deoxy-Sulfide Compound, Compound (4) may contain, after the oxidation, Deoxy-Sulfonyl Compound as an impurity.

Furthermore, since Deoxy-Sulfonyl Compound has properties closely similar to those of Compound (4), it is difficult to remove Deoxy-Sulfonyl Compound by isolating Compound (4) by the conventional procedure. Accordingly, in the present invention, the crude crystals of Compound (3) obtained after the synthesis of Compound (3) are purified (that is, Deoxy-Sulfide Compound is removed).

The purification of the crude crystals of Compound (3) is carried out by crystallization.

Examples of solvent used for the crystallization (hereinafter, referred to as Crystallization Solvent) includes Crystallization Solvent containing toluene or chlorobenzene, and the like, Crystallization Solvent containing toluene is preferred, Crystallization Solvent containing not less than 50% by weight of toluene is more preferred and Crystallization Solvent substantially consisting of toluene is particularly preferred.

The amount of Crystallization Solvent to be used is usually 4 to 7 ml and preferably 5 to 6 ml based on 1 g of Compound (3).

Compound (3) is dissolved in the above Crystallization Solvent with heating.
The temperature for dissolution with heating is usually 65°C to 80°C and preferably 65°C to 75°C.

Desirably, seed crystals (purified crystals of Compound (3)) are added in the crystallization step. The addition is usually carried out after the above dissolution with heating and before starting of the crystallization.

The addition of the seed crystals is preferred because it becomes possible to avoid adherence of the crystals to the wall of a container due to too rapid crystallization and to avoid uneven shape of crystals, and hence there is a tendency that subsequent filtration, washing and drying becomes easy.

In view of advantages that the crystals are surely formed, Compound (3) with stable quality is surely obtained and so on, the amount of the seed crystals to be added is usually 0.01 to 0.02% by weight and preferably 0.01 to 0.015% by weight based on the amount of Compound (3).

The temperature of the solution for receiving the seed crystals is usually 50 to 60°C and preferably 53 to 57°C.

After the addition of the seed crystals, the solution is subjected to maturing and cooling, if necessary, in the crystallization to stabilize its quality.

In the present invention, the maturing refers to standing of the solution containing Compound (3) at a temperature in a range as defined below, in order to promote growth of the crystals.

In the maturing, the solution is stirred at around 55°C, usually at 55 ±5°C and preferably at 55±2°C.

In the maturing, the stirring at around 55°C is advantageous because the quality is stabilized.

From viewpoints of crystallization of uniform crystals and of easy filtration, washing and drying, the period for maturing (period for continuation of stirring at a temperature around 55°C) is usually 1 to 3 hours, preferably 1 to 2 hours and more preferably about 1 hour.

In the cooling at crystallization, the solution is cooled from the maturing temperature (around 55°C), usually 55±5°C and preferably 55 ±2°C to around 10°C, usually 10±5°C and preferably 10±2°C.

Because of advantages that Compound (3) can be obtained in a stable yield and so on, the cooling rate in the cooling step is usually 5 to 15°C/hour, preferably 10 to 15°C/hour and more preferably about 10°C/hour

From a viewpoint of improvement of the yield, it is desirable to stir, after cooling, for additional 1 to 3 hours and preferably 1 to 2 hours at the same temperature.

Additionally, when the formed crystals are filtered, the crystals are washed with the same solvent as that for crystallization (preferably toluene) usually at 5 to 12°C and preferably 8 to 12°C.

The amount of the solvent to be used for the washing is usually 1.5 to 3.5 ml and preferably 2 to 3 ml based on 1 g of Compound (3).

The purified crystals of Compound (3) are obtained by such crystallization step.

The purification of Compound (3) is particularly effective when an amount exceeding 0.06% (LC area percentage) of Deoxy-Sulfide Compound based on the crude crystals of Compound (3) is contained.

By controlling the amount of Deoxy-Sulfide Compound contained in the purified crystals of Compound (3) to not more than 0.06% (LC area percentage) based on Compound (3), the amount of Deoxy-Sulfonyl Compound in the crystals of Compound (4) as the objective product can be controlled to less than 0.10% (LC area percentage) based on Compound (4). This effect has been first found by the present inventors, and Compound (4) containing less than 0.10% (LC area percentage) of Deoxy-Sulfonyl Compound is suitable as the pharmaceutical.

The purification of the crude crystals of Compound (3) by crystallization is applied to any crystals of Compound (3) containing 0.06 to 0.5%, preferably about 0.06 to 0.4% and more preferably about 0.06 to 0.3% of Deoxy-Sulfide Compound. Of course, no problem occurs when the purification is applied to crystals of Compound (3) containing not more than 0.06% of Deoxy-Sulfide Compound.

Compound (4) can be obtained by reacting Compound (3) with a peroxycarboxylic acid, using the purified crystals of Compound (3) obtained in the above purification (hereinafter, this step is referred to as Step C).

As the peroxycarboxylic acid, those exemplified for Step A can be used similarly and preferably include mono-perphthalic acid.

In Step C, peroxycarboxylic acid is added to Compound (3) in a suitable reaction solvent.

Preferred reaction solvent in the reaction of the step C is ethyl acetate from a viewpoint of operability.

The amount of the solvent to be used is usually 1 to 3 ml and preferably 1.5 to 2.5 ml based on 1 g of Compound (3).

The amount of peroxycarboxylic acid to be used is usually 3 to 5 moles and preferably 3.5 to 4.5 moles based on 1 mole of Compound (3).

With regard to the method for adding peroxycarboxylic acid, dropwise addition of a peroxycarboxylic acid solution is preferred from viewpoints of easiness of addition, safety and operability. When peroxycarboxylic acid solution is added dropwise, the solution may be added in two or more divided portions.

Examples of solvent suitable for preparing peroxycarboxylic acid solution include, ethyl acetate, ethers (for example, diethyl ether or the like) and so on, ethyl acetate is preferred from a viewpoint of safety. It is desirous to use the same solvent as the above solvent for reaction.

The amount of the solvent to be used for preparing peroxycarboxylic acid solution is usually 3 to 10 ml, preferably 3.5 to 7 ml, based on 1 g of peroxycarboxylic acid.

When peroxycarboxylic acid solution is added dropwise, the dropping rate depends on the concentration of the dropping solution, and the temperature of the dropping solution and drop-receiving solution, but is usually 1 to 4 ml/min and preferably 1.5 to 3 ml/min based on 1 g of Compound (3).

When peroxycarboxylic acid solution is added dropwise, the temperature of the dropping solution is usually 0 to 30°C and preferably 10 to 25°C.

When peroxycarboxylic acid solution is added dropwise, the temperature of the drop-receiving solution is usually 0 to 20°C and preferably 0 to 10°C.

The reaction temperature is usually 0 to 20°C and preferably 0 to 10°C.

The reaction period varies depending on the reaction temperature and other reaction conditions but is usually 0.5 to 5 hours and preferably 1 to 3 hours.

After the reaction, for example, a solvent (for example, an organic solvent such as ethyl acetate or the like) for extraction is added to the reaction mixture, and the mixture after addition of the solvent is stirred and settled. After settling, Compound (4) can be isolated by washing and concentrating the extract (organic layer) obtained by phase separation. If necessary, the obtained Compound (4) may be purified according to the conventional process (for example, process described in WO 03/053920 or the like) or a process similar thereto.

In the description, the LC area percentage (%) refers to a datum, each expressed in percentage, of a peak area for Deoxy-Sulfide Compound against Compound (3) or for Deoxy-Sulfonyl Compound against Compound (4), in a chromatogram obtained from analysis of LC (liquid chromatography, preferably HPLC (high performance liquid chromatography)).

Conditions for LC: SUMIPAX ODS A-212, acetonitrile/0.1% aqueous acetic acid solution.

### Example 1

Wet crystals of
4'-cyano-3-(4-fluorophenylthio)-2-hydroxy-2-methyl-3'-trifluoromethylpropionanilide (83.8 g; dry weight: 75.4 g) containing Deoxy-Sulfide Compound (0.19% (LC area percentage)) were added to toluene (377.1 ml), heated and dissolved. The solution was cooled down to 55°C. Seed crystals (10 mg) of
4'-cyano-3-(4-fluorophenylthio)-2-hydroxy-2-methyl-3'-trifluoromethylpropionanilide were added, and the solution was cooled to 50°C over an hour. Then, the solution was cooled to 10°C over 4 hours at a rate of 10°C/hour. After stirring for 2 hours at 10°C, the product was filtered and washed with toluene (226 ml) cooled to 10°C. Upon drying at 47 to 48°C under 2.4 to 2.7 kPa, purified crystals of
4'-cyano-3-(4-fluorophenylthio)-2-hydroxy-2-methyl-3'-trifluoromethylpropionanilide were obtained. The content of Deoxy-Sulfide Compound was 0.052% (LC area percentage). Conditions for HPLC: (SUMIPAX ODS A-212; acetonitrile/0.1% aqueous acetic acid solution).

### Example 2

Purified crystals of
4'-cyano-3-(4-fluorophenylthio)-2-hydroxy-2-methyl-3'-trifluoromethylpropionanilide (10.0 g, 25.1 mmol) (containing 0.061% (LC area percentage) of Deoxy-Sulfide Compound) obtained in the same manner as in the above Example 1, phthalic anhydride (18.64 g, 125.6 mmol) and ethyl acetate (30 ml) were charged and heated to 50°C.
Hydrogen peroxide (5.4 g, 55.6 mmol) was added dropwise over 8 hours and the mixture was matured for 2 hours. Ethyl acetate (90 ml) was added and the mixture was cooled to 5°C. Na₂SO₃ (0.71 g) was dissolved in deionized water (6.1 ml), and the solution was added dropwise. After confirming absence of the per-oxy acid, the mixture was heated to 15°C and 20% aqueous K₂CO₃ solution was added dropwise thereto for adjusting pH to 7.07. After washing with a solution prepared from NaCl (6.0 g), deionized water (60 ml) and K₂CO₃ (0.17 g), the solution (pH 7.58) was subjected to acid washing [35% HCl (1.5 g) and deionized water (50 ml)] (pH 0.60). The organic layer was combined with ethyl acetate (60 ml x 2) and concentrated under reduced pressure. Analysis of water revealed that the content was 0.77%. After adding activated carbon (0.51 g) and ethyl acetate (4.2ml), the mixture was heated to 70°C and filtrated. Seed crystals (10 mg) were added and the mixture was stirred at 50°C for 1 hour. The organic layer was concentrated under reduced pressure and then n-heptane (40 ml) was added dropwise thereto. After cooling to 7°C, the product was left to stand for 2 hours and filtered to give purified crystals of
4'-cyano-3-[(4-fluorophenyl)sulfonyl]-2-hydroxy-2-methyl-3'-trifluoromethylpropionanili de(9.69 g, yield: 89.7%). The content of Deoxy-Sulfonyl Compound was 0.094% (LC area percentage). Conditions for HPLC: (SUMIPAX ODS A-212; acetonitrile/0.1% aqueous acetic acid solution).

### Example 3

N-methacryloyl-4-cyano-3-trifluoromethylaniline (57.3 g, 0.225 mol), ethyl acetate (340 ml) and phthalic anhydride (116.9g, 0.789 mol) were charged and the mixture was heated to 50 to 55°C.

To the mixture was added dropwise 35% aqueous hydrogen peroxide solution (43.8 g, 0.451 mol) over 8 hours and the mixture was matured for 20 hours. After adding ethyl acetate (114 ml), the mixture was cooled to 15°C and then aqueous solution (145 ml) of sodium sulfite (25.6 g) was added dropwise thereto.

After confirming absence of the per-oxy acid, 15% aqueous potassium hydroxide solution was added dropwise to the mixture, for adjusting pH to 7.3. Phases were separated and a layer was washed with a solution prepared from water (230 ml), sodium chloride (40.5 g) and 35% aqueous hydrochloric acid (0.23 g).

After concentrating the organic layer under reduced pressure, toluene (400 ml) was added and the layer was further concentrated. The concentrate obtained was combined with THF (344 ml) and cooled down to 5°C. Then triethylamine (11.4 g, 0.113 mol) and subsequently methanesulfonyl chloride (6.5 g, 0.056 mol) were added dropwise and the mixture was matured at the same temperature for 30 minutes.

Then, a mixed solution of 4-fluorothiophenol (34.7 g, 0.27 mol) with toluene (28 ml), and subsequently triethylamine (11.4 g, 0.113 mol) were added dropwise, and the mixture was matured at the same temperature for 30 minutes. After heating to 50°C, the mixture was matured at the same temperature for 5 hours.

After cooling to 15°C, the mixture was washed with 135 g of 15% aqueous sodium chloride, then with a solution prepared from 143 ml of water, 19.5 g of sodium chloride and 7.8 g of sodium carbonate. At this point, the content of Deoxy-Sulfide Compound was 0.064% (LC area percentage).

After phase separation, the organic layer was concentrated, combined with 400 ml of toluene and concentrated further under reduced pressure.

After heating to 75°C, 69 ml of toluene, 2.9 g of alumina and 3.4 g of activated carbon were added and the mixture was stirred at the same temperature for 30 minutes. After filtering while hot, the filter was washed with 57 ml of toluene and the obtained organic layer was cooled down to 55°C. After crystallization by seeding with seed crystals (0.017% by weight), the mixture was matured at 56 to 50°C for an hour. Then, the mixture was cooled (13.3°C/hour) down to 10°C over 3 hours. After cooling, the mixture was matured at 10 ± 2°C for 2 hours.

Upon filtration and washing, 80.0 g of a wet cake of
4'-cyano-3-(4-fluorophenylthio)-2-hydroxy-2-methyl-3'-trifluoromethylpropionanilide was obtained.

After drying, 76.0 g (yield: 84.6%) of a dry cake was obtained.

The content of Deoxy-Sulfide Compound in the dry cake was not detected (n.d.).

To the purified and Deoxy-Sulfide Compound non-detected crystals of
4'-cyano-3-(4-fluorophenylthio)-2-hydroxy-2-methyl-3'-trifluoromethylpropionanilide was added Deoxy-Sulfide Compound, separately prepared, at ratios (LC area percentage (%)) shown in Table 1. Using the obtained crystals of 4'-cyano-3-(4-fluorophenylthio)-2-hydroxy-2-methyl-3'-trifluoromethylpropionanilide, the experiments were conducted in the same manner as in Example 2 to give crystals of
4'-cyano-3-[(4-fluorophenyl)sulfonyl]-2-hydroxy-2-methyl-3'-trifluoromethylpropionanili de. The results are shown in Table 1.

**Table 1**

| Deoxy-sulfide compound % (LC area percentage) | Deoxy-sulfonyl compound % (LC area percentage) | Yield of compound (I) (%) |
|---|---|---|
| N.D. | 0.036 | 88.4 |
| 0.034 | 0.068 | 85.3 |
| 0.040 | 0.068 | 84.4 |
| 0.052 | 0.076 | 85.2 |
| 0.061 | 0.094 | 89.7 |
| 0.070 | 0.118 | 49.7 |

### Example 4

N-methacryloyl-4-cyano-3-trifluoromethylaniline (1 part by weight), ethyl acetate (5.4 parts by weight) and phthalic anhydride (2 parts by weight) were charged and the mixture was heated to 50 to 55°C.

To the mixture was added dropwise 35% aqueous hydrogen peroxide solution (0.57 part by weight) over 9.7 hours and the mixture was matured for 22.3 hours. After adding ethyl acetate (1.8 parts by weight), the mixture was cooled to 15°C and then aqueous solution (1 part by weight) of sodium sulfite (0.17 part by weight) was added dropwise thereto.

After confirming absence of the per-oxy acid, 15% aqueous potassium hydroxide solution was added dropwise to the mixture, for adjusting pH to 7.0. Phases were separated and a layer was washed with a solution prepared from water (4 parts by weight), sodium chloride (0.7 part by weight) and 35% aqueous hydrochloric acid (0.004 part by weight).

After concentrating the organic layer under reduced pressure, toluene (6 parts by weight) was added and the layer was further concentrated. The concentrate was combined with THF (5.3 parts by weight) and cooled down to 5°C. Then triethylamine (0.08 parts by weight) and subsequently methanesulfonyl chloride (0.045 parts by weight) were added dropwise and the mixture was matured at the same temperature for 30 minutes.

Then, a mixed solution of 4-fluorothiophenol (0.6 part by weight) with toluene (0.43 part by weight), and subsequently triethylamine (0.2 part by weight) were added dropwise, and the mixture was matured at the same temperature for 30 minutes. After heating to 50°C, the mixture was matured at the same temperature for 6 hours.

After cooling to 15°C, the mixture was washed with a solution prepared from water (2 parts by weight), sodium chloride (0.14 part by weight) and sodium carbonate (0.35 part by weight). At this point, the content of Deoxy-Sulfide Compound was 0.22% (LC area percentage).

After phase separation, the organic layer was concentrated, combined with toluene (8.8 parts by weight) and concentrated further under reduced pressure.

After heating to 75°C, toluene (1 part by weight), alumina (0.05 part by weight) and activated carbon (0.06 part by weight) were added and the mixture was stirred at the same temperature for 30 minutes.

After filtering while hot, the filter was washed with toluene (0.9 part by weight) and the obtained organic layer was cooled down to 55°C. After crystallization by seeding with seed crystals (0.017% by weight), the mixture was matured at 54±1°C for an hour. Then, the mixture was cooled (5°C/hour) down from 55°C to 50°C over 1 hour and cooled (7.4°C/hour) down from 50°C to 10°C over 5.4 hours. After cooling, the mixture was matured at 10±2°C for 2 hours.

Upon filtration and washing, a wet cake of
4'-cyano-3-(4-fluorophenylthio)-2-hydroxy-2-methyl-3'-trifluoromethylpropionanilide (1.49 parts by weight) was obtained. After drying, a dry cake (1.33 parts by weight, yield: 85.0%) was obtained.

The content of Deoxy-Sulfide Compound was 0.035% (LC area percentage).

The obtained dry cake (1 part by weight) of
4'-cyano-3-(4-fluorophenylthio)-2-hydroxy-2-methyl-3'-trifluoromethylpropionanilide, ethyl acetate (2.7 parts by weight) and phthalic anhydride (1.86 parts by weight) were charged and heated to 50 to 55°C.

To the mixture was added dropwise 35% aqueous hydrogen peroxide solution (0.54 part by weight) over 9.6 hours and the mixture was matured for 2 hours. After adding ethyl acetate (8.1 parts by weight), the mixture was cooled to 5°C and then 10% aqueous solution of sodium sulfite (0.8 part by weight) was added dropwise thereto.

After confirming absence of the per-oxy acid, the mixture was heated to 15°C, 20% aqueous sodium hydroxide solution (9.7 parts by weight) was added dropwise to the mixture for adjusting pH to 6.9. Phases were separated and a layer was washed with a solution prepared from water (5 parts by weight) and 35% hydrochloric acid (0.0027 part by weight). The organic layer was combined with ethyl acetate (10.8 parts by weight) and ethyl acetate (10.3 parts by weight) was evaporated by concentration. Further ethyl acetate (2.4 parts by weight) was charged and ethyl acetate (2.8 parts by weight) was evaporated by concentration. Then, ethyl acetate (0.38 part by weight) and activated carbon (0.5 part by weight) were charged and the mixture was stirred at 70 to 75°C. The mixture was filtered while hot, and the filter was washed with ethyl acetate (0.9 part by weight). After cooling down to 55°C, crystallization by seeding with seed crystals was carried out. The mixture was cooled down to 7°C and matured at the same temperature for 2 hours.

Filtration and washing gave a wet cake (1.14 parts by weight) of
4'-cyano-3-[(4-fluorophenyl)sulfonyl]-2-hydroxy-2-methyl-3'-trifluoromethylpropionanili de. After drying, a dry cake (0.975 part by weight; yield: 85.8%) was obtained. The content of Deoxy-Sulfonyl Compound was 0.073% (LC area percentage).

### Reference Example

An example showing the difficulty of removal of Deoxy-Sulfonyl Compound from Compound (4) is shown below.

To ethyl acetate (78 ml) was added
4'-cyano-3-[(4-fluorophenyl)sulfonyl]-2-hydroxy-2-methyl-3'-trifluoromethylpropionanili de (4.80 g) (containing 0.118% (LC area percentage) of Deoxy-Sulfonyl Compound), and the mixture was heated to 75°C and dissolved. Activated carbon (Shirasagi A1) (0.23 g) and ethyl acetate (2 ml) were added and the mixture was stirred for 30 minutes at 70 to 75°C and filtered. After gradually cooling to 50°C, seed crystals (10mg) were added and the mixture was stirred at 50°C for an hour. The organic layer was decreased by about 30 g by concentration under reduced pressure, and n-heptane (20 ml) was added dropwise to the mixture. After cooling down to 7°C, the mixture was stirred for 2 hours.

### Filtration gave

4'-cyano-3-[(4-fluorophenyl)sulfonyl]-2-hydroxy-2-methyl-3'-trifluoromethylpropionanili de (4.08 g; yield: 85%; Deoxy-Sulfonyl Compound: 0.102% (LC area percentage)).

According to the present invention, Compound (4) which is useful medicine as an anticancer drug can be provided in higher quality (impurity of less than 0.10%) and by an industrially advantageous way.

## Claims

1. A process for producing bicalutamide of the formula (4); 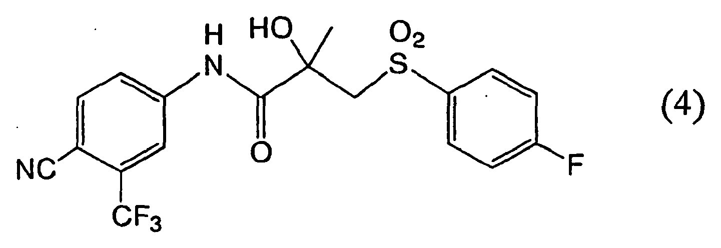 which comprises
Step A comprising reacting Compound (1) of the formula (1); 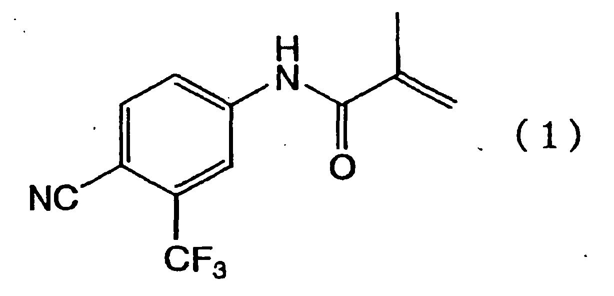 with peroxycarboxylic acid to obtain Compound (2) of the formula (2); 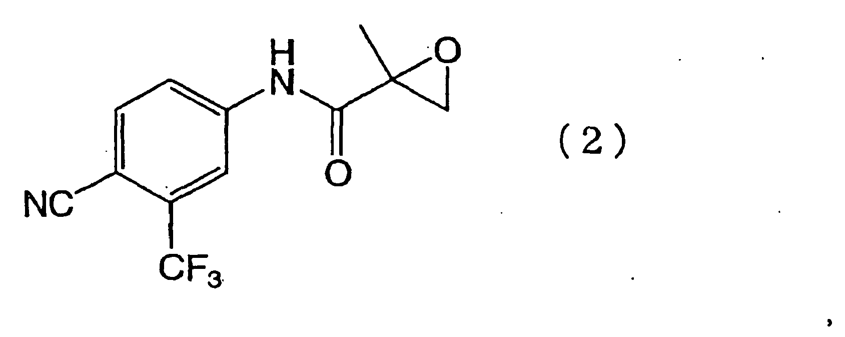
Step B comprising reacting said Compound (2) with 4-fluorothiophenol to obtain crude crystals of Compound (3) of the formula (3); 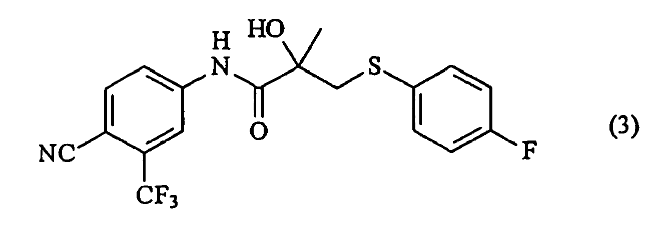 comprising 0.06 to 0.5% of 4'-cyano-3-(4-fluorophenylthio)-2-methyl-3'-trifluoromethylpropionanilide, dissolving the crude crystals in a solvent and crystallizing to obtain purified crystals of Compound (3), and
Step C comprising reacting Compound (3) and percarboxylic acid to obtain bicalutamide.

2. The process according to Claim 1, wherein the solvent comprises toluene.

3. The process according to Claim 2, wherein the solvent consists essentially of toluene.

4. The process according to any one of the preceding Claims, wherein seed crystals of purified Compound (3) are added before starting the crystallization.

5. The process according to any one of the preceding Claims, wherein the crystallization comprises maturing and cooling.

6. The process according to Claim 5, wherein the maturing is conducted at a temperature of 55 ± 5°C.

7. The process according to Claim 5 or 6, wherein the time of maturing is 1 to 2 hours.

8. The process according to Claim 5,6 or 7, wherein the cooling is conducted from 55°C to 10°C.

9. The process according to Claim 5, 6, 7 or 8, wherein the speed of cooling is 5 to 15 °C/hour.

## Patentansprüche

1. Ein Verfahren zur Herstellung von Bicalutamid der Formel (4); 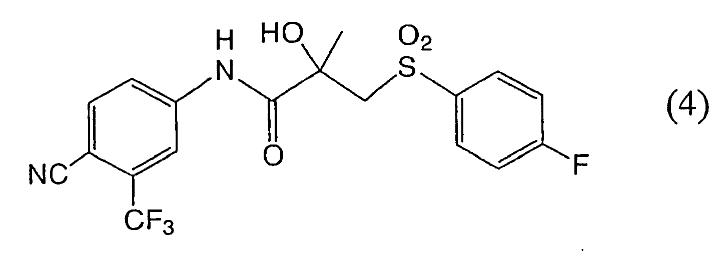 welches umfasst:
den Schritt A, umfassend das Umsetzen der Verbindung (1) der Formel (1); 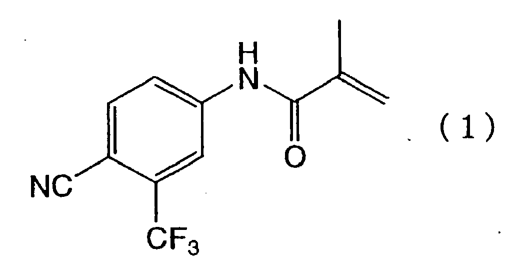 mit einer Peroxycarbonsäure, um die Verbindung (2) der Formel (2) zu erhalten; 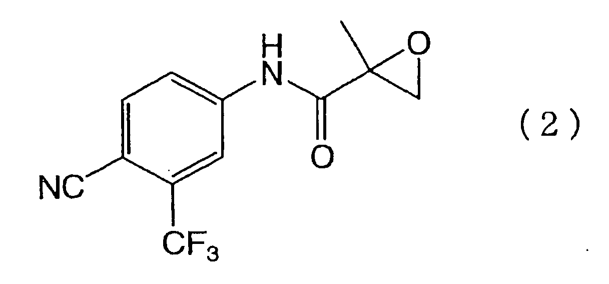
den Schritt B, umfassend das Umsetzen der Verbindung (2) mit 4-Fluorthiophenol, um Rohkristalle der Verbindung (3) der Formel (3) zu erhalten; 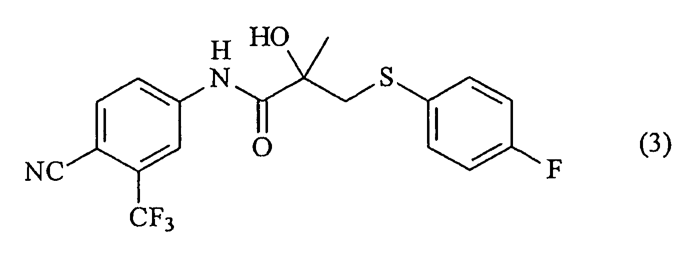 umfassend 0,06 bis 0,5% 4'-Cyano-3-(4-fluorphenylthio)-2-methyl-3'-trifluormethyl-propionanilid, Auflösen der Rohkristalle in einem Lösungsmittel und Kristallisieren, um gereinigte Kristalle der Verbindung (3) zu erhalten, und
den Schritt C, umfassend das Umsetzen der Verbindung (3) und einer Percarbonsäure, um Bicalutamid zu erhalten.

2. Das Verfahren gemäß Anspruch 1, wobei das Lösungsmittel Toluol umfasst.

3. Das Verfahren gemäß Anspruch 2, wobei das Lösungsmittel im Wesentlichen aus Toluol besteht.

4. Das Verfahren gemäß einem der vorstehenden Ansprüche, wobei Impfkristalle der gereinigten Verbindung (3) vor Beginn der Kristallisation hinzugefügt werden.

5. Das Verfahren gemäß einem der vorstehenden Ansprüche, wobei die Kristallisation Reifung und Abkühlen umfasst.

6. Das Verfahren gemäß Anspruch 5, wobei das Reifen bei einer Temperatur von 55 ± 5°C durchgeführt wird.

7. Das Verfahren gemäß Anspruch 5 oder 6, wobei die Reifungszeit 1 bis 2 Stunden beträgt.

8. Das Verfahren gemäß Anspruch 5, 6 oder 7, wobei das Abkühlen von 55°C auf 10°C durchgeführt wird.

9. Das Verfahren gemäß Anspruch 5, 6, 7 oder 8, wobei die Geschwindigkeit des Abkühlens 5 bis 15°C/Stunde beträgt.

## Revendications

1. Procédé de production de bicalutamide de la formule (4) : 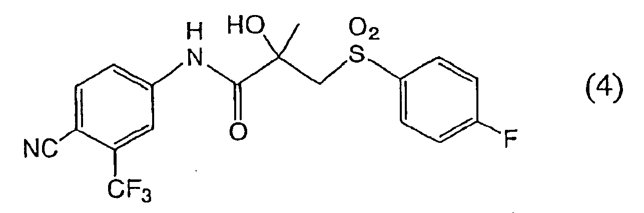 lequel comprend
l'étape A comprenant la réaction du composé (1) de la formule (1) : 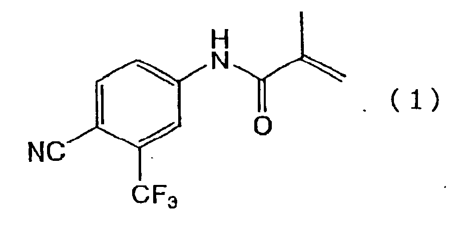 avec de l'acide peroxycarboxylique pour obtenir le composé (2) de la formule (2) : 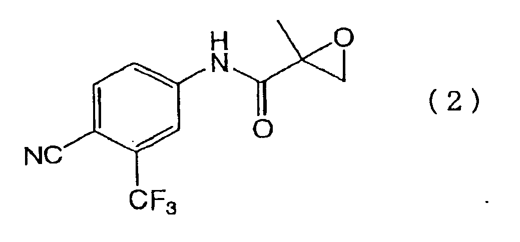
l'étape B comprenant la réaction dudit composé (2) avec du 4-fluorothiophénol pour obtenir des cristaux bruts de composé (3) de la formule (3) : 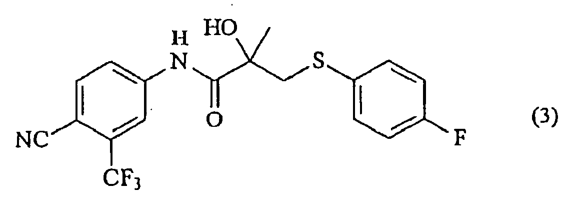 comprenant de 0,06 à 0,5 % de 4'-cyano-3-(4-fluorophénylthio)-2-méthyl-3'-trifluorométhylpropionanilide, en dissolvant les cristaux bruts dans un solvant et en les cristallisant pour obtenir des cristaux purifiés de composé (3), et
l'étape C comprenant la réaction du composé (3) et d'acide percarboxylique pour obtenir du bicalutamide.

2. Procédé selon la revendication 1, dans lequel le solvant comprend du toluène.

3. Procédé selon la revendication 2, dans lequel le solvant est essentiellement constitué de toluène.

4. Procédé selon l'une quelconque des revendications précédentes, dans lequel des cristaux de germes de composé purifié (3) sont ajoutés avant le début de la cristallisation.

5. Procédé selon l'une quelconque des revendications précédentes, dans lequel la cristallisation comprend la maturation et le refroidissement.

6. Procédé selon la revendication 5, dans lequel la maturation est réalisée à une température de 55 ± 5°C.

7. Procédé selon la revendication 5 ou 6, dans lequel la durée de maturation est de 1 à 2 h.

8. Procédé selon la revendication 5, 6 ou 7, dans lequel le refroidissement est réalisé à de 55°C à 10°C.

9. Procédé selon la revendication 5, 6, 7 ou 8, dans lequel la vitesse de refroidissement est de 5 à 15°C/h.
